Europäisches Patentamt

European Patent Office

Office européen des brevets

(11) Publication number: **0 023 942**

**A1**

# EUROPEAN PATENT APPLICATION

(21) Application number: **79710002.1**

(22) Date of filing: **10.08.79**

(51) Int. Cl.³: **A 61 G 9/00**
**A 61 F 5/44**

(43) Date of publication of application:
**18.02.81 Bulletin 81/7**

(84) Designated Contracting States:
**CH DE FR GB SE**

(71) Applicant: **Horiuchi, Akira**
**12-10, Akatsuka Shinmachi, 1-chome Itabashi-ku**
**Tokyo-to(JP)**

(72) Inventor: **Horiuchi, Akira**
**12-10, Akatsuka Shinmachi, 1-chome Itabashi-ku**
**Tokyo-to(JP)**

(74) Representative: **Zipse, Erich et al,**
**Patentanwälte Zipse + Habersack Kemnatenstrasse 49**
**D-8000 München 19(DE)**

(54) **Help implement to be used by human females when urinating.**

(57) Some help implements for urinating require the female to assume an inconvenient posture others are inconvenient for domestic use because their washing and sterilisation are troublesome. The present help implement is made of thick paper or like in a compact size, so that the implement can be manufactured at a low cost and therefore readily disposed of.

It has a gourd shaped body (1) with, at a lower oval portion (5), an inner peripheral wall (6) gently curved back so as to define a passage opening at the outlet end of which a cylindrical connector (8) is connected.

0023942

PATENTANWÄLTE     — 1 —     Telefon (089) 170186

# ZIPSE + HABERSACK

Baden-Baden   München     Telegramme LILOPAT

Kemnatenstraße 49

**D-8000 MÜNCHEN 19**

DIPL.-ING. H.-J. HABERSACK
DIPL.-PHYS. E. ZIPSE     KI 1-EP

08.08.1979

Akira Horiuchi
Tokyo-to   Japan

Help implement to be used by human
females when urinating

The present invention relates to a help implement to be used by human females when urinating. The implement of the present invention is particularly designed for use by human females under conditions where difficulties are encountered with respect to urination. Thus, a woman confined to a sickbed finds it difficult to urinate conveniently. Also, women who are out on camping trips or are in other situations where facilities such as water closets are unavailable find it difficult to urinate conveniently. In the case of a female individual who is confined to a sickbed, there are conventional urinals available, but these generally require the individual to assume a posture where she is seated on the urinal with the hips and knees suitably bend in an extremely uncomfortable position. Under conditions where an ill woman cannot assume a posture of this latter type as, for example, when the illness necessitates complete rest without the possibility of deviating from a prone position, especially difficult conditions are encountered. Or the particular individual may suffer from a disease related to nerves, bones or muscles or deformation of the lumbar vertebrae, with the result that it is possible for the individual in this latter type of situation to be supported by a plaster cast or other fixing device so that it may be possible even with difficulty only to use a portable urinal of small size. Such urinals have conventionally, without exception, taken the form of a relatively large container of glass or synthetic resin having an urine inlet projecting therefrom. With the use of such a conventional urinal the patient still is required to extend both thighs at a relatively wide angle with the lower part of the body exposed, so that there frequently is a heightening of the sense of shame and a spell of psychological uneasiness particularly with respect to such factors as, for example, whether or not the particular individual can properly urinate into such a conventional urinal without soiling the bed. Such a sense of shame and psychological disturbance have sometimes caused dysuria. In view of the above considerations there is a serious requirement for an instrument for enabling urination to be carried out conveniently and without any difficulty by a patient in a prone position while eliminating any sense of shame or psychological uneasiness.

The inventor has developed, to meet such serious requirement, a help implement capable of being effectively and conveniently used by an ill female in a prone position, without in any way disturbing the rest of such an individual so that even if such an individual cannot assume the natural posture required for urination nevertheless it will be possible to urinate conveniently, as disclosed in Swiss Patent No. 566 774. According to this patent,the help implement includes an elongated body having a relatively large inlet end and an opposed substantially smaller outlet end with is adapted to be connected with a tube for conveying urine beyond the outlet end of the body. The body gradually diminishes in cross section from its relatively large inlet end to its opposed outlet end, and the body is formed with a passage extending from the inlet to the outlet end of the body longitudinally through the body. The body is formed of glass or synthetic resin. When the implement is used one end of a tube of rubber, synthetic resin, or the like is connected to the smaller end of the body, and the other end of this tube can be connected with a well known urinal or other urine receiving receptacle. In the case of an individual who is confined to bed, such a tube may extend to a urinal situated on the floor beside the bed. The relatively large end of the body is positioned by the user so that this relatively large end covers the cavity inside the labium minus pudendi, including the ostium urethrae externum and the vestibulum vaginae(vestibule of the vagina). In this way it is possible to position the end of the passage at the relatively large end of the body so that it coincides with the ostium urethrae externum, thus assuring that urine discharging from the urethra will be directly received in the passage of the body to flow through this passage into the tube connected to the relatively small end of the body.

Once accustomed to using, this help implement to be used by human females when urinating which has already been developed by

- 4 -

the inventor is extremely convenient, but this must be subjected to washing and sterilization after use, so that such implement is certainly convenient for use in hospital or like well furnished with the equipment for such washing and sterilization but somewhat inconvenient for domestic use because said washing and sterilization are troublesome.

In view of such aspect the inventor provides according to the present invention an improved and simplified help implement to be used by human females when urinating, which is adapted to be readily disposed of after each use, well maintaining the advantages of the previously developed implement as mentioned above.

The present invention provides a help implement to be used by human females when urinating comprising a substantially gourd-shaped body formed of material such as thick paper including an upper relatively small oval portion having a shallow recess formed therein and a lower relatively large oval portion having a peripheral wall gradually curved back so as to define an opening at the rear end and a cylindrical connector adapted to be connected with said opening.

The present invention will be now described more in detail with reference to a preferred embodiment illustrated by the accompanying drawing.

Fig. 1 is a perspective view showing the help implement to be used by human females when urinating;

Fig. 2 is a front view corresponding to Fig. 1; and

Fig. 3 is a longitudinal section corresponding to Fig. 1.

The implement shown is formed by press working and preferably subjected to waterproofing treatment. This implement may be formed of, instead of said thick paper, laminated combination of synthetic resin or paper and synthetic resin film which is similar to thick paper.

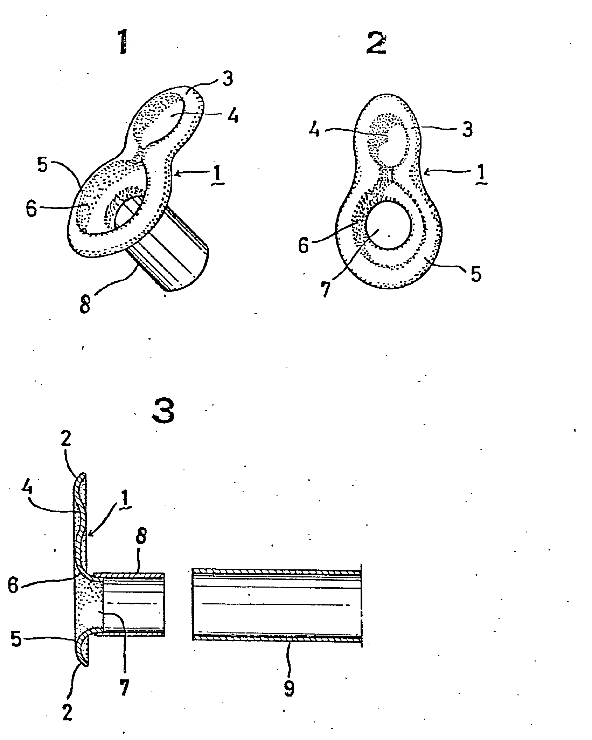

As it will be obvious from Fig. 2, a body 1 substantially gourd-shaped as seen in front view is stamped out from thick paper and the whole periphery thereof is preferably curved back in a limited extent to form a curve 2. An upper relatively small oval portion 3 of the body 1 is formed of a shallow recess 4 and a lower relatively large oval portion 5 is gradually curved back to form a curved peripheral wall 6 defining an opening 7 at the rear end thereof, to which a cylindrical connector 8 is connected. With the embodiment shown, the cylindrical connector 8 also is formed of thick paper and integrated with the body 1 by suitable adhesive means. Obviously the body 1 and the cylindrical connector 8 may be formed of material similar to thick paper such as synthetic resin and, in this case, the body 1 and the cylindrical connector 8 may be molded in unison. Reference numeral 9 designates a conduit leading to the urinal or the urine-receiving receptacle and this conduit 9 also is preferably formed of thick paper or like.

When the help implement to be used by human females when urinating of the arrangement as aforementioned, the body 1 is longitudinally positioned with the shallow recess 4 formed in the upper oval portion being applied to the clitoris(glans clitoridis) included in the pupendum femininum so that the opening 7 defined by the curved peripheral wall 6 correctly covers the ostium urethrae externum. In this way, the labium minus pudendi closely enclose the body 1 along the periphery thereof, assuring a stable positioning thereof in use.

After positioning in this manner, urine can be discharged through the conduit 9 into the urinal or the urine-receiving receptacle. Use of the implement according to the present invention makes it possible for an ill female in a prone position to urinate conveniently without in any way disturbing the rest of such an individual and without the sense of shame and a spell of psychological uneasiness. Particularly, the implement is made of thick paper or like in a compact size and a simplified arrangement according to the present invention, so that the implement can be manufactured at a low cost and therefore readily disposed of. The last-mentioned feature assures a high hygiene and advantageously makes it possible for this implement to be safely used in average home poorly furnished with the equipment for washing and sterilization.

## Claims

1. A help implement to be used by human females when urinating comprising a body having a passage extending from an inlet to an outlet end of the body, the inlet end of the body being substantially gourd shaped and including an upper relatively small oval portion having a shallow recess formed therein facilitating positioning of the body at a location where the passage at a lower relatively large oval portion of the inlet end of the body ist adapted to receive urine from the urethra, c h a r a c t e r i z e d in that the gourd s-haped body (1) is press formed of thick paper, synthetic resin, a laminated combination thereof or the like and the lower relatively large oval portion (5) of the body having an inner peripheral wall (6) gently curved back so as to define a passage opening (7) and a cylindrical connector (8) being connected with the outlet end of said opening.

2. A help implement according to claim 1, c h a rac t e r i z e d in that the whole periphery thereof is curved back in a limited extent to form a curve (2).

3. A help implement according to claim 1 or 2, c h a r a c t e r i z e d in that said cylindrical connector (8) also is formed of thick paper or the like and integrated with the body (1) by suitable adhesive means.

4. A help implement according to claim 1 or 2, c h a rac t e r i z e d in that said body (1) and said connector (8) are formed of a material such as synthetic resin to be molded in unison.

5. A help implement according to one of claims 1 - 4, c h a r a c t e r i z e d by a conduit (9) also formed of thick paper or the like and adapted to be connected with the connector (8).

6. A help implement according to  one of claims 1-5,
c h a r a c t e r i z e d  in that when formed of thick paper,
it is subjected to a waterproofing treatment.

0023942

**European Patent Office**

**EUROPEAN SEARCH REPORT**

Application number

EP 79 71 0002

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| D,X | CH - A5 - 566 774 (HORIUCHI et al.) <br> * column 1, lines 62 to 65; column 3, lines 41 to 42; column 5, lines 19 to 24 and 32 to 34; fig. 1, 2b, positions 29, 17, 20, 14, 15, 28 * <br> & US - A - 3 864 759 <br> & FR - A - 2 189 002 <br> --- | 1,2, 4,5 | A 61 G 9/00 <br> A 61 F 5/44 |
| | CH - A5 - 591 238 (LI) <br> * claims 8 to 12; column 1, lines 40 to 45 and 61 to 67 * <br> --- | 6 | **TECHNICAL FIELDS SEARCHED (Int.Cl.3)** <br><br> A 61 F 5/44 <br> A 61 G 9/00 |
| | GB - A - 2 002 629 (TOWFIGH) <br> * whole document * <br> --- | 6 | |
| A | DE - A - 2 249 132 (EAST/WEST MEDICAL PRODUCTS) <br> * fig. 11, 12, position 118' * <br> --- | 1 | |
| A | GB - A - 1 467 144 (HOLLISTER INC.) <br> * fig. 1, position 14 * <br> ---- | 1 | |

| X | The present search report has been drawn up for all claims | | |
|---|---|---|---|
| Place of search | Date of completion of the search | Examiner | |
| Berlin | 09-04-1980 | DROPMANN | |

EPO Form 1503.1 06.78